# EUROPEAN PATENT APPLICATION

(11) **EP 3 766 544 A1**
(43) Date of publication of application: **20.01.2021**
(21) Application number: 19305954.0
(22) Date of filing: 18.07.2019
(51) Int. Cl.: A61P 35/00, A61P 39/06, C07D 231/06, C07D 401/04, A61K 31/4155

(54) **NOVEL PYRAZOLONE DERIVATIVES AS PD-1/PD-L1 INTERACTION INHIBITORS**

(71) Applicant: Centre Hospitalier Regional Universitaire de Lille, 59000 Lille (FR); Université de Lille, 59800 Lille (FR); INSTITUT NATIONAL DE LA SANTE ET DE LA RECHERCHE MEDICALE - INSERM, 75013 Paris (FR)
(72) Inventor: THURU, Xavier, 59300 AULNOY LEZ VALENCIENNES (FR); QUESNEL, Bruno, 59000 LILLE (FR); MAGNEZ, Romain, 59800 LILLE (FR); MILLET, Régis, 62440 HARNES (FR); LELEU, Natascha, 59147 GONDECOURT (FR); KLUPSCH, Frédérique, 62410 HULLUCH (FR)
(74) Representative: Regimbeau

(57) **Abstract**

The present invention relates to novel pyrazolone derivatives of the following formula (I): 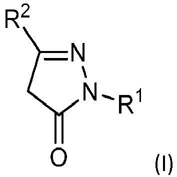 or a pharmaceutically acceptable salt and/or solvate thereof and to a pharmaceutical composition comprising thereof.

The present invention also concerns the use of compound of formula (I) or the pharmaceutical composition comprising said compound as a drug, notably in the prevention and/or treatment of PD-L1-PD-1 interactions-related diseases, such as cancer, neurological diseases and chronic infections.

The present invention also relates to a method for the preparation of a compound of formula (I).

## Description

### Field of the invention

The present invention relates to novel pyrazolone derivatives which act advantageously as PD-1/PD-L1 interaction inhibitors in the prevention and/or treatment of cancer, neurological diseases and chronic infections, to pharmaceutical compositions comprising said pyrazolones and to a process for their preparation.

### Background of the invention

Tumor dormancy results from a long-term equilibrium between dormant tumor cells and host (Adv Exp Med Biol. 2013;734:181-200) . Several factors contribute to dormant tumor cells immunoescape. Among them, a protein called PD-L1 (aliases B7-H, B7H1, PDCD1L1, CD274, PDCD1LG1, PDL1 and hPD-L1) plays an important role in positive and negative regulation of T-lymphocyte-mediated immune response (Immunity. 2018 Mar 20;48(3):434-452). Indeed, PD-L1 (Programmed death-ligand 1) primarily inhibits T-cells via its interaction with the PD-1 receptor, on the T-cell surface, and through direct interaction with the B7.1 (also called CD80) molecule (Nat Rev Immunol. 2018 Mar;18(3):153-167). B7.1 can also directly inhibit T-lymphocytes via the CTLA4 inhibitory receptor expressed on the T-cell surface. Several experimental studies have demonstrated that these interactions between PD-L1 and PD-1, between PD-L1 and B7.1, and between B7.1 and CTLA4 may have a role in limiting the antitumor immune response in situations where immune responses against cancer cells play an essential role (Nat Med. 2002;8:793-800, Nat Med. 2003;9:562-567). One of those situations is tumor dormancy (APMIS. 2008 Jul-Aug;116(7-8):685-94).

There is now evidence that patients in complete remission can carry cancer cells for very long periods - in some cases indefinitely - without relapsing (Nat Cell Biol. 2018 Nov;20(11):1240-1249). The phenomenon of long-term persistence of cancer cells that do not grow is called tumor dormancy. Tumor dormancy is commonly observed clinically, and in many cancers, there are late relapses after years or decades of complete remission. Few experimental models of tumor dormancy are available, and dormant tumor cells in humans are difficult to isolate, making scientific progress relatively slow. However, several experimental models have led to the discovery of some mechanisms of dormancy. The inventors have previously demonstrated, by using animal models, that a balance exists between host antitumor immunity and dormant tumor cells and that these cells develop specific immunoescape mechanisms (Gene Ther. 2000 Aug;7(15):1312-6, Blood. 2004 Oct 1;104(7):2124-33, Blood. 2005 Mar 15;105(6):2428-35, Cancer Res. 2007 May 1;67(9):4491-8).

In particular, it was shown that, over time, dormant tumor cells become less sensitive to lysis and more resistant to specific cytotoxic lymphocytes (CTL)-mediated killing (Blood. 2004 Oct 1;104(7):2124-33). This gradual development of resistance suggests that the tumor does not merely survive passively, but that there is a continuous struggle between the host immune response and the dormant tumor cells. To allow relapse, any equilibrium between tumor cells and the immune system must break down. In the model designed by the inventors, dormant tumor cells escape the immune response by overexpressing PD-L1. PD-L1 inhibits CTL-mediated killing and allows long-term persistence of dormant tumor cells. A possible contributing factor is B7.1. B7.1, another member of the B7 family molecule, may inhibit T-cell through CTLA4 and also through interaction in cis with PD-L1. (Cancer Immunol Res; 6(8) August 2018). The inventors observed in their model of tumor dormancy that both PD-L1 and B7.1 contribute to tumor dormancy and that blocking either PD-L1, B7.1, or both molecules, inhibit tumor growth (Quesnel B., Saudemont A., Blood, 2004, 104, 2124-2133).

In summary, B7-1/PD-L1/PD-1/CTLA4 interactions play a key role on immunoescape in many cancers. Blocking these interactions should favor eradication of tumor cells through host immune responses, especially in the clinical situation of tumor dormancy where residual cancer cells are few. PD-L1/PD-1 interaction appears as the preferential target for cancer immunotherapy, as PD-L1 expression is more widely distributed in tumor cells than B7.1 which is expressed only on a limited number of hematological malignancies (Blood. 2013 Jan 31;121(5):734-44).

Several clinical studies (Nat Rev Cancer. 2019 Mar;19(3):133-150, Nat Rev Cancer. 2015 Aug;15(8):457-72) investigating monoclonal antibodies (mAbs) blocking PD-L1/PD-1 interactions have demonstrated dramatic complete response (CR) rate and overall survival (OS) improvement in malignant melanoma, lung cancer, Hodgkin's lymphoma, MMR colon cancer and several other tumor types. Complete response corresponds to the disappearance of all signs of cancer in response to treatment. However, these trials mainly include patients with a large tumor mass. In addition, mAbs showed an important toxicity, their effects lasting for weeks, increasing the risk of severe autoimmune responses. Indeed, PD-L1/PD-1 interactions are physiological regulatory mechanisms that protect the body against inappropriate immune responses. Another limitation is the poor bioavailability of mAbs, which do not penetrate profoundly inside tumor masses (Proc Natl Acad Sci USA. 2015 Nov 24;112(47):E6506-14).

There is thus a need for compounds with better bioavailability, able to penetrate deeper into tumors, and whose effects would be quickly reversible after the interruption of the treatment.

Small molecules targeting PD-L1/PD-1/B7.1/CTLA4 would be able to act inside tumors, and their effects would be rapidly reversible by drug withdrawal in the case of severe adverse events.

The inventors have thus developed small molecules that block the interaction between PD-L1/PD-1 to allow restoration of the host's antitumor immune response against dormant tumor cells and, more generally, to tumors in which PD-L1 participate in immunoescape. Pyrazolone derivatives have been identified as having a strong blocking potential of the different interactions between PD-L1/PD-1. These molecules are aimed to be used to block immunoescape mechanisms in patients in cancer remission, allowing eradication or at least long-term control of minimal residual disease.

### Summary of the invention

The present invention relates, in a first aspect, to compound of the following formula (I): 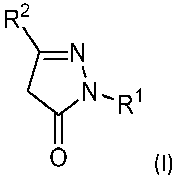 or a pharmaceutically acceptable salt and/or solvate thereof,
wherein
R¹ is selected in the group consisting of: 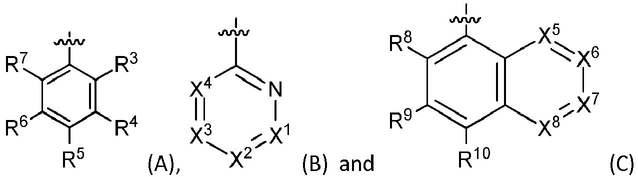
X¹ is N or CR¹¹, X² is N or CR¹², X³ is N or CR¹³, X⁴ is N or CR¹⁴, X⁵ is N or CR¹⁵, X⁶ is N or CR¹⁶, X⁷ is N or CR¹⁷ and X⁸ is N or CR¹⁸, provided that at least two of X¹ to X⁴ are not N and that at least two of X⁵ to X⁸ are not N,
R³ to R¹⁸ are each independently selected in the group consisting of H, halogen, such as Cl or F, CN, COR¹⁹ and OR²⁰, R¹⁹ and R²⁰ being each independently H or C₁-C₆ alkyl,
R² is selected in the group consisting of carbocyclyl, heterocyclyl, aryl and heteroaryl, R² being optionally substituted by one or more groups selected in the group consisting of halogen, such as Cl or F, NO₂, C₁-C₆ haloalkyl, such as CF₃, oxo, CN, SO₂ and OR²¹, R²¹ being H or C₁-C₆ alkyl.

In a second aspect, the present invention relates to a pharmaceutical composition comprising at least one compound of formula (I) and at least one pharmaceutically acceptable excipient.

In a third aspect, the present invention relates to a compound of formula (I) according to the invention or a pharmaceutically acceptable salt and/or solvate thereof, or a pharmaceutical composition according to the present invention for use as a drug, in particular in the prevention and/or treatment of PD-L1-PD-1 interactions-related diseases, such as cancer, neurological diseases and chronic infections.

The present invention also relates to the use of a compound of formula (I) according to the invention or a pharmaceutically acceptable salt and/or solvate thereof, or a pharmaceutical composition according to the present invention for the manufacture of a drug, notably intended in the prevention and/or treatment PD-L1-PD-1 interactions-related diseases, such as cancer, neurological diseases and chronic infections.

The present invention also relates to the use of a compound of formula (I) according to the invention or a pharmaceutically acceptable salt and/or solvate thereof, or a pharmaceutical composition according to the present invention for the prevention and/or treatment of PD-L1-PD-1 interactions-related diseases, such as cancer, neurological diseases and chronic infections.

The present invention also relates to a method for the prevention and/or treatment of PD-L1-PD-1 interactions-related diseases, such as cancer, neurological diseases and chronic infections, comprising the administration to a person in need thereof of an effective dose of a compound of formula (I) according to the invention or a pharmaceutically acceptable salt and/or solvate thereof, or a pharmaceutical composition according to the present invention.

In a fourth aspect, the present invention concerns a method of preparation of compounds of formula (I) according to the invention.

### Definitions

The term "stereoisomers" used in this invention refers to configurational stereoisomers and more particularly to optical isomers.

In the present invention, the optical isomers result from the different position in space of substituents or lone pair of electrons on an atom (such as a carbon atom) comprising four different substituents (including potentially a lone pair of electrons). This atom thus represents a chiral or asymmetric center. Optical isomers that are not mirror images of one another are thus designated as "diastereoisomers", and optical isomers, which are non-superimposable mirror images are designated as "enantiomers".

An equimolar mixture of two enantiomers of a chiral compound is designated as a racemic mixture or racemate.

The term "tautomers" refers to structural isomers, or constitutional isomers, resulting from the relocation of a proton in the molecule. Contrarily to stereoisomers in which only spatial arrangement differs, in structural isomers, the molecule has different bonding patterns and atomic organization.

For example, tautomers of the compound of formula (I) in equilibrium can be illustrated as below:

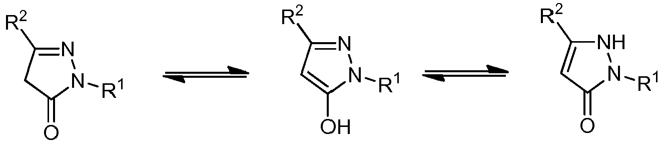

For the purpose of the invention, the term "pharmaceutically acceptable" is intended to mean what is useful to the preparation of a pharmaceutical composition, and what is generally safe and nontoxic, for a pharmaceutical use.

The term "pharmaceutically acceptable salt and/or solvate" is intended to mean, in the framework of the present invention, a salt and/or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L25 tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

The term "halogen", as used in the present invention, refers to a fluorine, bromine, chlorine or iodine atom.

The term "C₁-C₆ alkyl", as used in the present invention, refers to a straight or branched monovalent saturated hydrocarbon chain containing from 1 to 6 carbon atoms including, but not limited to, methyl, ethyl, n-propyl, iso-propyl, n-butyl, iso-butyl, sec-butyl, *t*-butyl, n-pentyl, n-hexyl, and the like.

The term "C₁-C₆ haloalkyl" refers to a C₁-C₆ alkyl chain as defined above wherein one or more hydrogen atoms are replaced by a halogen atom selected from fluorine, chlorine, bromine or iodine, preferably a fluorine atom. For example, it is a CF₃ group.

The term "carbocyclyl" refers to a non-aromatic hydrocarbon ring, saturated or unsaturated, typically comprising from 3 to 20 carbons and comprising one or more fused or bridged ring(s). In particular, it is a saturated hydrocarbon cycle, especially a C₃-C₇ cycloalkyl, including, but not limited to, cyclopropyl, cyclobutyl, cyclopentyl, cyclohexyl, cycloheptyl, and the like.

The term "C₃-C₇ cycloalkyl" refers to a saturated hydrocarbon ring comprising from 3 to 7 carbons, including cyclopropyl, cyclobutyl, cyclopentyl, cyclohexyl and cycloheptyl.

The term "heterocyclyl" as used in the present invention refers to a non-aromatic, saturated or unsaturated monocycle or polycycle (comprising fused, bridged or spiro rings) comprising preferably 5 to 10, notably 5 or 6, atoms in the ring(s), in which the atoms of the ring(s) consist of carbon atoms and one or more, advantageously 1 to 4, and more advantageously 1 or 2, heteroatoms, such as a nitrogen, oxygen or sulphur atom, the remainder being carbon atoms. A heterocycle can be notably piperidinyl, piperizinyl, pyrrolidinyl, pyrazolidinyl, imidazolidinyl, azepanyl, thiazolidinyl, isothiazolidinyl, oxazocanyl, thiazepanyl, benzimidazolonyl.

The term "aryl" refers to an aromatic hydrocarbon group preferably comprising from 6 to 12 carbon atoms and comprising one or more fused rings, such as, for example, a phenyl or naphthyl group. Advantageously, it is a phenyl group.

The term "heteroaryl", as used in the present invention, refers to an aromatic group comprising one or several, notably one or two, fused hydrocarbon cycles in which one or several, notably one to four, advantageously one or two, carbon atoms each have been replaced with a heteroatom selected from a sulfur atom, an oxygen atom and a nitrogen atom, preferably selected from an oxygen atom and a nitrogen atom. It can be a furyl, thienyl, pyrrolyl, pyridyl, oxazolyl, isoxazolyl, thiazolyle, isothiazolyl, imidazolyl, pyrazolyl, oxadiazolyl, thiadiazolyl, triazolyl, tetrazolyl, pyridazinyl, pyrimidinyl, pyrazinyl, triazinyl, quinolyl, isoquinolyl, quinoxalyl or indyl.

In the context of the present invention, "unsaturated" means that the hydrocarbon chain may contain one or more unsaturation(s), i.e. a double bond C=C or a triple bond C≡C, advantageously one.

The term "pharmaceutical composition" is meant in the framework of the present invention a composition having preventive and curative properties towards cancers.

According to the invention, the expression "PD-L1-PD-1 interactions-related diseases" includes any disease due to the inhibition of T-cell activation, notably caused by the interaction between PD-L1 and PD-1 and the treatment or prevention of which would benefit from the use of an inhibitor of said interaction. For example, PD-L1-PD-1 interaction-related diseases includes cancer, neurological disease and chronic infection.

The term "radiotherapy" as used in the present invention, refers to a method of treatment, notable for treating cancer, using radiations to kill disease cells.

The term "virotherapy" as used in the present invention, refers to a therapeutic disease using a virus, such as an adenovirus, an adeno-associated virus or a retrovirus or other virus after reprogramming it by means of genetic engineering. In the field of cancer and tumor control, an adenovirus can be reprogrammed to specifically target cancer cells and tissues to kill them.

### Detailed description

### Compound of formula (I)

The compounds according to the present invention can be in the form of a tautomer, a stereoisomer or a mixture of stereoisomers in any proportions thereof, in particular a mixture of enantiomers, and notably a racemic mixture.

In compound of formula (I), R³ to R¹⁸ are advantageously each independently selected in the group consisting of H, halogen, such as Cl or F, CN and OR²⁰, R²⁰ being H or C₁-C₆ alkyl. Preferably, R³ to R¹⁸ are each independently selected in the group consisting of H, halogen, such as Cl or F and OR²⁰, R²⁰ being preferably a C₁-C₆ alkyl such as a methyl.

According to a particular embodiment, compound of formula (I) corresponds to the following formula (I-A): 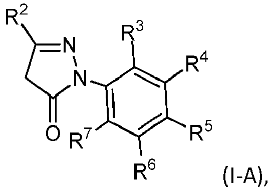 wherein R¹ is of formula (A).

According to this embodiment, R³ to R⁷ are advantageously each independently selected in the group consisting of H, halogen, such as Cl and OR²⁰, R²⁰ being preferably a C₁-C₆ alkyl such as a methyl. In particular, two of R³ to R⁷ are halogen, such as Cl, and the others are H.

According to another particular embodiment, compound of formula (I) corresponds to the following formula (I-B): 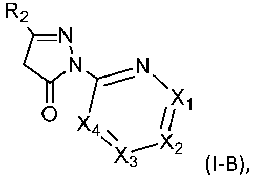 wherein R¹ is of formula (B).

According to this embodiment, R¹¹ to R¹⁴ are preferably H or halogen, such as Cl, in particular H.

Advantageously, in compound of formula (I-B), at least three of X¹ to X⁴ are not N. More preferably, none of X¹ to X⁴ is N.

More advantageously, X¹ is CR¹¹, X² is CR¹², X³ is CR¹³ and X⁴ is CR¹⁴ and R¹¹ to R¹⁴ are H.

According to another particular embodiment, compound of formula (I) corresponds to the following formula (I-C): 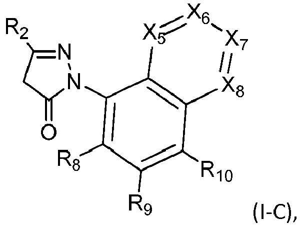 wherein R¹ is of formula (C).

According to this embodiment, R¹⁵ to R¹⁸ are preferably H or halogen, such as Cl, in particular H.

Advantageously, in compound of formula (I-C), one or two, preferably one, of X⁵ to X⁸ are/is N.

In compound of formula (I), R² is preferably selected in the group consisting of carbocyclyl and aryl, R² being optionally substituted by one or more groups selected in the group consisting of halogen, such as Cl or F, NO₂, C₁-C₆ haloalkyl, such as CF₃, oxo, CN, SO₂ and OR²¹, R²¹ being H or C₁-C₆ alkyl.

In a preferred embodiment, R² is selected in the group consisting of carbocyclyl and aryl, such as phenyl, R² being optionally substituted by one or more groups, preferably one or two, selected in the group consisting of halogen, such as Cl or F, NO₂, C₁-C₆ haloalkyl, such as CF₃, oxo and OR²¹, R²¹ being C₁-C₆ alkyl, such as methyl.

In particular, R² is an aryl, notably a phenyl. According to this embodiment, R² may be not substituted. Preferably, R² is substituted by one or more groups, preferably one or two, selected in the group consisting of halogen, such as Cl, NO₂, C₁-C₆ haloalkyl, such as CF₃, and OR²¹, R²¹ being preferably a methyl.

According to another particular embodiment R² is a carbocyclyl. According to this embodiment, R² is preferably not substituted. For example, R² may be a C₃-C₇-cycloalkyl, such as a cycloheptyl. R² may also be a carbocyclyl comprising briged and/or fused rings. Notably, R² corresponds to the following adamantyl ring:

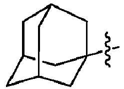

According to a preferred embodiment, in compounds of formula (I), R¹ is of formula (A) or (B). In particular, R¹ is of formula (A) or (B) and R² is selected in the group consisting of carbocyclyl and aryl, R² being optionally substituted by one or more groups, preferably one or two, selected in the group consisting of halogen, such as Cl or F, NO₂, C₁-C₆ haloalkyl, such as CF₃, oxo, CN, SO₂ and OR²¹, R²¹ being H or C₁-C₆ alkyl.

More particularly, R¹ is of formula (A), wherein R³ to R⁷ are each independently selected in the group consisting of H, halogen, such as Cl and OR²⁰, R²⁰ being preferably a C₁-C₆ alkyl such as a methyl, notably R³ to R⁷ are H or halogen and R² is a carbocyclyl, such as adamantyl.

According to a preferred embodiment, the present invention relates to the following compounds of formula (I): 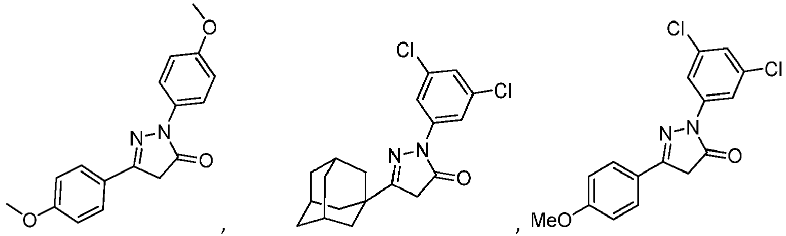 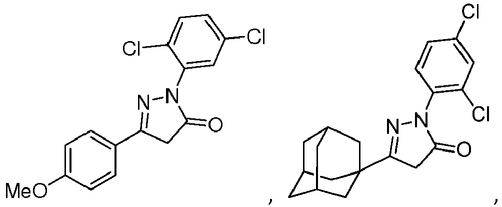 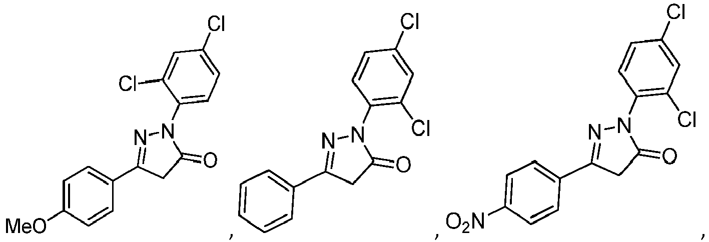 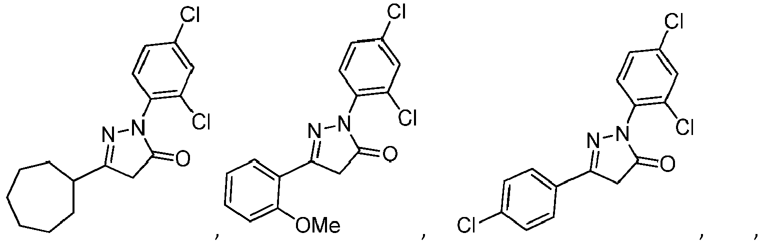 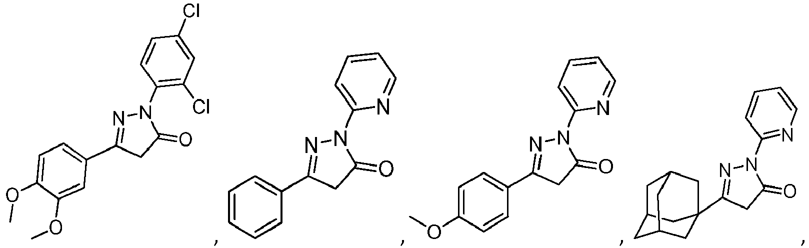 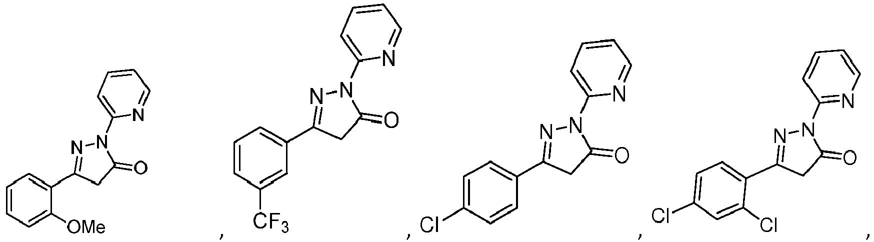 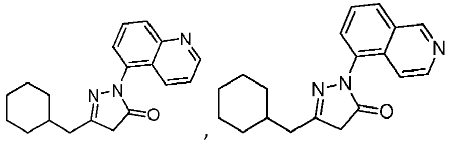 preferably 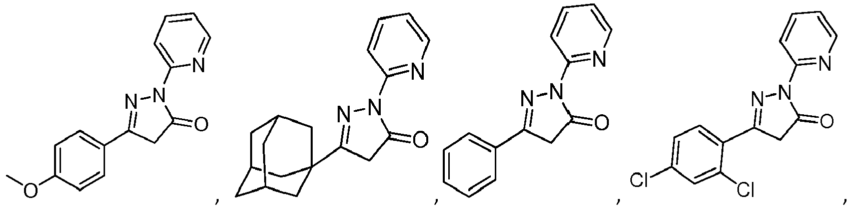 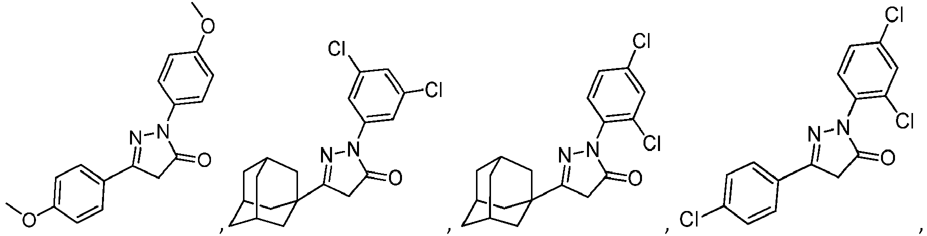 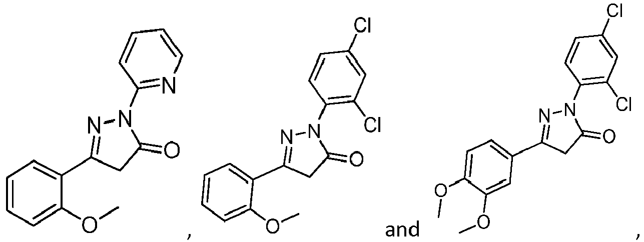 more preferably 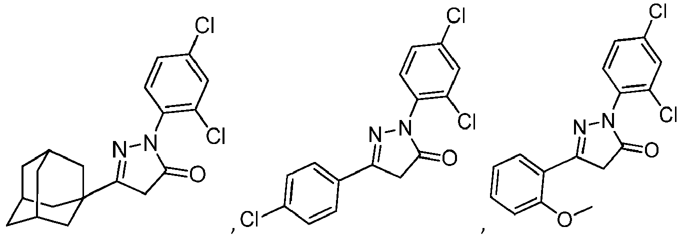 and

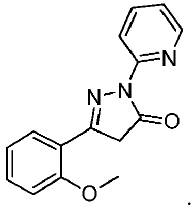

### Pharmaceutical composition

The present invention also relates to a pharmaceutical composition comprising at least one compound of formula (I) or a pharmaceutically acceptable salt and/or solvate thereof, and at least one pharmaceutically acceptable excipient.

The pharmaceutical compositions of the invention can be intended to oral or parenteral (e.g. subcutaneous, intramuscular, intravenous) administration, preferably oral or intravenous administration. The active ingredient can be administered in unit forms for administration, mixed with conventional pharmaceutical carriers, to animals, preferably mammals including humans.

For oral administration, the pharmaceutical composition can be in a solid or liquid (solution or suspension) form.

A solid composition can be in the form of tablets, gelatin capsules, powders, granules and the like. In tablets, the active ingredient can be mixed with pharmaceutical vehicle(s) such as gelatin, starch, lactose, magnesium stearate, talc, gum arabic and the like before being compressed. The tablets may be further coated, notably with sucrose or with other suitable materials, or they may be treated in such a way that they have a prolonged or delayed activity. In powders or granules, the active ingredient can be mixed or granulated with dispersing agents, wetting agents or suspending agents and with flavor correctors or sweeteners. In gelatin capsules, the active ingredient can be introduced into soft or hard gelatin capsules in the form of a powder or granules such as mentioned previously or in the form of a liquid composition such as mentioned below.

A liquid composition can contain the active ingredient together with a sweetener, a taste enhancer or a suitable coloring agent in a solvent such as water. The liquid composition can also be obtained by suspending or dissolving a powder or granules, as mentioned above, in a liquid such as water, juice, milk, etc. It can be for example a syrup or an elixir.

For parenteral administration, the composition can be in the form of an aqueous suspension or solution which may contain suspending agents and/or wetting agents. The composition is advantageously sterile. It can be in the form of an isotonic solution (in particular in comparison to blood).

The compounds of the invention can be used in a pharmaceutical composition at a dose ranging from 0.01 mg to 1000 mg a day, administered in only one dose once a day or in several doses along the day, for example twice a day in equal doses. The daily administered dose is advantageously comprised between 5 mg and 500 mg, and more advantageously between 10 mg and 200 mg. However, it can be necessary to use doses out of these ranges, which could be noticed by the person skilled in the art.

The pharmaceutical compositions according to the invention may further comprise at least one other active ingredient, such as an anticancer agent. In particular, the anticancer agent may be an anti-CTLA4 antibody such as Ipilimumab, a CAR-T compound such as axicabtagene ciloleucel, an anti-LAG3 antibody such as BMS-986016, an anti-TIM3 antibody such as MBG453, an antiCD47 antibody such as Hu5F9-G4, a small molecule blocking SIRP1α, an anti-VISTA antibody, an anti-TIGIT antibody, an anti CD200 antibody such as Samalizumab, an anti-CD38 antibody such as daratumumab, an anti-TNFa inhibitor such as Etanercept, and an anti-200R inhibitor such as OX2 inhibitory peptide.

### Treatment

Compound of formula (I) is notably useful as inhibitors of the PD-L1/PD-1 interaction. It is especially able to target dormant tumor cells.

Accordingly, compound of formula (I) according to the invention or a pharmaceutically acceptable salt and/or solvate thereof, or a pharmaceutical composition according to the present invention is useful as a drug, in particular in the prevention and/or treatment of PD-L1-PD-1 interactions-related diseases, such as cancer, neurological diseases and chronic infections.

The present invention relates also to a pharmaceutical composition comprising:
(i) at least one compound of formula (I) or a pharmaceutically acceptable salt and/or solvate thereof as defined above, and
(ii) at least one other active ingredient, such as an anticancer agent,
as a combination product for simultaneous, separate or sequential use, in particular in the prevention and/or treatment of PD-L1-PD-1 interactions-related diseases, such as cancer, neurological diseases and chronic infections.

According to a particular embodiment, cancer may be selected from lung carcinoma (Non-small cell and Small cell), head and neck carcinoma, bladder carcinoma, kidney carcinoma, triple negative breast cancer, melanoma, gastric carcinoma, oesophagus carcinoma, Hodgkin's lymphoma, non-Hodgkin lymphoma, glioblastoma, multiple myeloma, acute myeloid leukemia, Acute Lymphoblastic Leukemia, Cholangiocarcinoma (gallbladder carcinoma), Merkel carcinoma, squamous cell carcinoma and endometrial carcinoma.

Neurological diseases may be for example Alzheimer disease.

Chronic infections may be selected from HIV, malaria, tuberculosis and B hepatitis.

Compound of formula (I) according to the invention or a pharmaceutically acceptable salt and/or solvate thereof, or a pharmaceutical composition according to the present invention may be used in the prevention and/or treatment of PD-L1-PD-1 interactions-related diseases, such as cancer, neurological diseases and chronic infections, in association with radiotherapy and/or virotherapy.

### Method of preparation

The present invention also relates to a method of preparation of compound of formula (I) or pharmaceutically acceptable salt and/or solvate thereof, said method comprising reacting a β-cetoester derivative of the following formula (II): 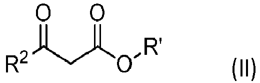 wherein R² is as defined above and R' is a C₁-C₆ alkyl,
with a hydrazine hydochloride derivative of the following formula (III): 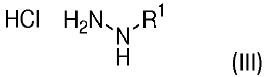 in presence of a base.

Advantageously in the compound of formula (II), R' is an ethyl.

According to a preferred embodiment, the reaction of compound of formula (II) with compound of formula (III) is achieved in a polar solvent, in particular a protic polar solvent such as acetic acid, alcohol and water. Preferably, the solvent is acetic acid.

The base is in particular a weak base such sodium acetate.

Compounds of formula (II) and (III) can be prepared by methods well-known from the skilled person in the art.

### Description of the figures

**Figure 1****:** Schematic representation of the FRET principle: Without FRET phenomenon, the excited fluorochrome emits its own fluorescence (left). Since there is a second fluorochrome close to the first, energy transfer can be made, which results in an excitation of the first fluorochrome (donor, named CFP) and an emission of the second fluorochrome (acceptor, named YFP) (right).
**Figure 2****:** ITC assay between ligand ALIPD 11 and the target rhB7-H1 Fc Chimera. (a) Raw heat plot, (b) Integration heat plot.

### Examples

### General procedure for the synthesis of pyrazolones

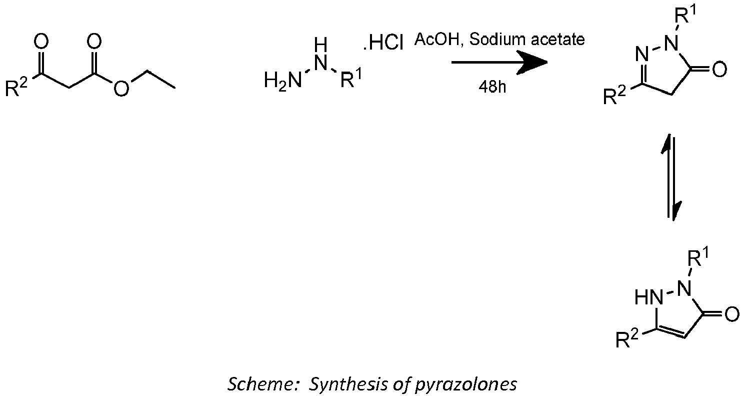

To a solution of β-cetoester (1 eq, 1 mmol) in AcOH (5 mL) were added sodium acetate (0.5 eq) and hydrazine hydrochloride (1eq). The reaction mixture was stirred for 48h at room temperature. After reaction, the solvent was evaporated under reduced pressure. The residue was dissolved in ethyl acetate (10 mL) and washed with water (10 mL) and brine (10 mL). The organic layer was dried over MgSO₄ and evaporated under reduced pressure.

### Biological results

**Table 1: Results of MST, FRET, ITC and in vitro spread assays for compounds of the invention**

| Compound | Structure | Mol. weight (g.mol⁻¹) | MST: K_{D} | FRET: IC₅₀ | ITC: K_{D} | *In vitro spread test* |
|---|---|---|---|---|---|---|
| **Nivolumab** | **C₆₃₆₂H₉₈₆₂N₁₇₁₂O₁₉₉₅S₄₂** | 143600 | 20 ± 2 nM | 14 ± 4 nM | - | - |
| **ALIPD55** | 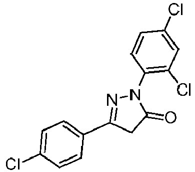 | 339,60 | 7 ± 3 nM | 3 ± 2 nM | - | - |
| **ALIPD11** | 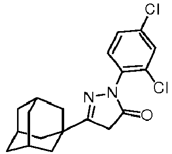 | 363,28 | 19 ± 3 nM | 9 ± 2 nM | 177 nM | 54 nM |
| **ALIPD60** | 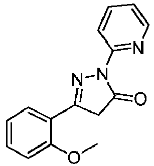 | 267,28 | 23 ± 5 nM | 7 ± 4 nM | - | - |
| **ALIPD51** | 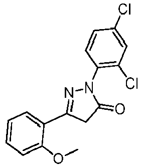 | 335,18 | 45 ± 7 nM | 44 ± 9 nM | - | - |
| **ALIPD19** | 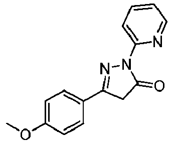 | 267,28 | 83 ± 12 nM | 17 ± 5 nM | - | 75 nM |
| **ALIPD26** | 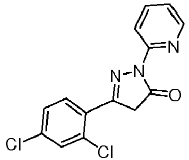 | 306,15 | 110 ± 17 nM | 98 ± 12 nM | - | - |
| **ALIPD52** | 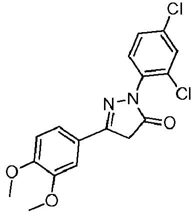 | 365,21 | 212 ± 23 nM | 196 ± 17 nM | - | - |
| **ALIPX57** | 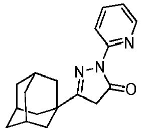 | 295,38 | 340 ± 27 nM | 4 ± 2 nM | - | 192 nM |
| **ALIPD24** | 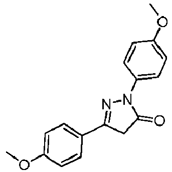 | 296,32 | 375 ± 43 nM | 230 ± 35 nM | - | No effect |
| **ALIPD53** | 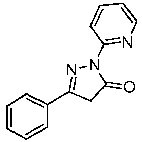 | 237,26 | 385 ± 21 nM | 359 ± 17 nM | - | - |
| **ALIPD28** | 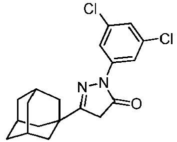 | 363,28 | 890 ± 75 nM | 785 ± 87 nM | - | - |
| **ALIPD58** | 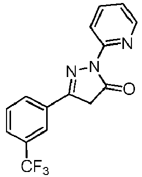 | 305,25 | 1,2 ± 0,2 µM | 1,1 ± 0,2 µM | - | - |
| **ALIPX14** | 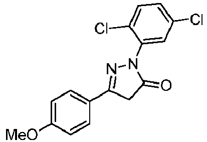 | 335,18 | 8 ± 2 µM | 12 ± 3 µM | - | - |
| **ALIPD48** | 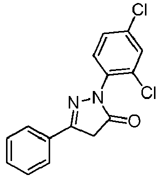 | 305,16 | 14 ± 3 µM | 9 ± 5 µM | - | - |
| **ALIPX02** | 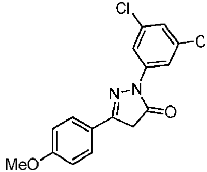 | 335,18 | 18 ± 3 µM | 15 ± 5 µM | - | - |
| **ALIPD59** | 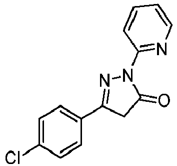 | 271,10 | 27 ± 5 µM | 33 ± 7 µM | - | - |
| **ALIPX05** | 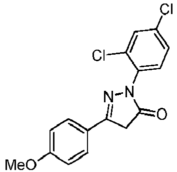 | 335,18 | 68 ± 7 µM | 51 ± 3µM | - | - |
| **ALIPD33** | 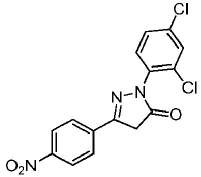 | 350,16 | 72 ± 17 µM | 43 ± 6 µM | - | - |
| **ALIPD38** | 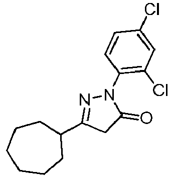 | 325,23 | 80 ± 12 µM | 67 ± 9 µM | - | - |

| | | | | | | |
|---|---|---|---|---|---|---|
| **MST** = **Microscale thermophoresis** **FRET** = Forster resonance energy transfer, i.e. **energy transfer between fluorescent molecules** **ITC** = **Isothermal titration calorimetry** | | | | | | |

### Comments:

Compounds are listed by affinity from nM to µM and compared to the reference antibody (Nivolumab) using Microscale Thermophoresis. Those physicochemical affinities are correlated by a functional FRET assay. Protocols are described below.

### Characterization of the compounds

### 1-(2,5-Dichlorophenyl)-3-(4-chlorophenyl)-4,5-dihydro-1H-5-pyrazolone (ALIPD55):

The product was recrystallized with acetonitrile. White solid. Yield: 37 %. mp: 196 °C. Rf (cyclohexane / ethyl acetate 6:4): 0,87. 1H NMR (DMSO): 5.99 (s, 1H); 7,45 (d, 2H, J=8,5 Hz); 7,57 (m, 2H); 7,77 (d, 2H, J=8,6 Hz); 7,85 (m, 1H); 11,65 (s, 1H). LC-MS (ESI+): 341,1 (MH+); tr= 3.18 min

### 3-(Adamantan-1-yl)-1-(2-pyridyl)-4,5-dihydro-1H-5-pyrazolone (ALIPD11):

The product was recrystallized with absolute ethanol. Beige solid. Yield: 31 %. mp: 218 °C Rf (cyclohexane / ethyl acetate 8:2): 0, 25. 1H RMN (DMSO): 1,70-1,98 (m, 15H); 5,33 (s, 1H); 7,43 (d, 1H, J=8,5 Hz); 7,50 (dd, 1H, J=8,4 Hz, J=2,3 Hz); 7,76 (d, 1H, J=2,2 Hz); 11,08 (s, 1H). LC-MS (ESI+): 363,2 (MH+); tr= 3,50 min.

### 3-(2-methoxyphenyl)-1-(2-pyridyl)-4,5-dihydro-1H-5-pyrazolone (ALIPD60):

The product was purified by flash chromatography on silica gel with eluent: cyclohexane / ethyl acetate (10:0) (7:3). White solid. Yield: 82 %. mp: 134 °C. Rf (cyclohexane / ethyl acetate 8:2): 0,36. ¹H NMR (CDCl₃): 3,94 (s, 3H); 6,18 (s, 1H); 7,02-7,09 (m, 2H); 7,16-7,19 (m, 1H); 7,34-7,40 (m,1H); 7,86-7,87 (m, 1H); 7,90-8,02 (m, 1H); 8,06-8,09 (d, 1H, *J*=8,4 Hz); 8,29-8,31 (d, 1H, *J*=4,3 Hz); 12,80 (s, 1H). LC-MS (APCl⁺): 268 (MH⁺); tᵣ=2,84 min.

### 1-(2,5-Dichlorophenyl)-3-(2-methoxyphenyl)-4,5-dihydro-1H-5-pyrazolone (ALIPD51):

The product was purified by chromatography on silica gel with the eluent: cyclohexane / ethyl acetate (8:2). The product was recrystallized with absolute ethanol. Beige solid. Yield: 53 %. mp: 128 °C. Rf (cyclohexane / ethyl acetate 6:4): 0,30. ¹H NMR (DMSO): 3,86 (s, 3H); 6,02 (s, 1H); 6;95 (t, 1H, *J*=7,4 Hz); 7,11 (d, 1H, *J*=8.2 Hz); 7,28 (t, 1H, *J*=7.2 Hz); 7,56 (s, 2H); 7,83 (m, 2H); 11,35 (s, 1H). LC-MS (ESI⁺): 335.1 (MH⁺); tᵣ= 2.93 min.

### 3-(4-Methoxyphenyl) -1-(pyridin-2-yl)-4,5-dihydro-1H-5-pyrazolone (ALIPD19):

The product was recrystallized with cyclohexane. Beige solid. Yield: 21 %. mp: 121°C. Rf (cyclohexane / ethyl acetate 1:1): 0,72. 1H NMR (CDCl3): 3,86 (s, 3H); 5,89 (s, 1H); 6,98 (d, 2H, J=8,9 Hz); 7,16 (t, 1H, J=6,3 Hz); 7,81 (d, 2H, J=8,9 Hz); 7,89 (t, 1H, J=7,4 H); 8,04 (d, 1H, J=8,4 Hz); 8,28 (d, 1H, J=6,1 Hz); 12,83 (s, 1H). LC-MS (ESI+): 268,2 (MH+); tr= 2,93 min.

### 3-(2,4-Dichlorophenyl)-1-(pyridin-2-yl)-4,5-dihydro-1H-5-pyrazolone (ALIPD26):

The product was recrystallized with absolute ethanol. White solid. Yield: 47 %. mp: 136°C. Rf (cyclohexane / ethyl acetate 6:4):0,51. 1H NMR (DMSO): 6,13 (s, 1H); 7,41 (t, 1H, J=5,6 Hz); 7,51 (dd, 1H, J=8,4 Hz, J=2,0 Hz); 7,72 (d, 1H, J=1,9 Hz); 7,86 (m, 2H); 8,07 (t, 1H, J=7,1 Hz); 12,44 (s,1H). LC-MS (ESI+): 306,1 (MH+); tr= 3,68 min.

### 1-(2,4-Dichlorophenyl)-3-(3,4-dimethoxyphenyl)-4,5-dihydro-1H-5-pyrazolone (ALIPD52):

The product was recrystallized with absolute ethanol. White solid. Yield: 22 %. mp: 223 °C. Rf (cyclohexane / ethyl acetate 1:1): 0,43. ¹H NMR (DMSO): 3.78 (s, 6H); 5.90 (s, 1H); 6,96 (d, 1H, *J*=8,25 Hz); 7,29 (m, 2H); 7,57 (m, 2H); 7,84 (m, 1H). LC-MS (ESI⁺): 365.1 (MH⁺); tᵣ= 2, 77 min.

### 3-(Adamantan-1-yl)-1-(pyridin-2-yl)-4,5-dihydro-1H-5-pyrazolone (ALIPX57):

The product was recrystallized with absolute ethanol. White solid. Yield: 73 %. mp: 163-164 °C. Rf (cyclohexane / ethyl acetate 6:4): 0,68. ¹H NMR (CDCl₃): 1,78-2,07 (m, 15H); 5,48 (s, 1H); 7,08 (t, 1H, *J*=6,4 Hz); 7,84 (dd, 1H, *J*=7,3 Hz, *J*=1,7 Hz); 7,91 (d, 1H, *J*=8,5 Hz); 8,23 (d, 1H, *J*=4,4 Hz). LC-MS (APCl⁺): 296,3 (MH⁺); tᵣ= 4,52 min.

### 1-(4-methoxyphenyl)-3-(4-methoxyphenyl)-4,5-dihydro-1H-5-pyrazolone (ALIPD24):

The product was recrystallized with absolute ethanol. Beige solid. Yield: 78 %. mp: 140°C. Rf (cyclohexane / ethyl acetate 8:2): 0,18. ¹H NMR (DMSO): 3,78 (s, 6H); 5,89 (s, 1H); 6;95 (d, 2H, *J*=8,7 Hz); 7,02 (d, 2H, *J*=9,0 Hz); 7,63 (t, 2H, *J*=9.0 Hz); 7,70 (d, 2H, *J*=8,8 Hz); 11,52 (s, 1H). LC-MS (ESI⁺): 297,2 (MH⁺); tᵣ= 2.77 min.

### 3-Phenyl-1-(2-pyridyl)-4,5-dihydro-1H-5-pyrazolone (ALIPD53):

The precipitate obtained was filtered and washed with ethanol (Fraction 1: yield 60 %). The filtrate was recovered and concentrated at reduced pressure. A solid was obtained which was recrystallized with ethanol (Fraction 2: yield 25 %). Pale yellow solid. Yield: 85 %. mp: 118 °C. Rf (cyclohexane / ethyl acetate 8:2): 0,4. ¹H NMR (CDCl₃): 5,86 (s, 1H); 7,16-7,20 (m, 1H); 7,34-7,46 (m, 3H); 7,86-7,93 (m, 3H); 8,06-8,09 (d, 1H, *J*=8,4 Hz); 8,28-8,30 (d, 1H, *J*=4,3 Hz); 12,79 (s, 1H). LC-MS (APCl⁺): 238 (MH⁺); tᵣ=3,10 min.

### 3-(Adamantan-1-yl)-1-(3,5-dichlorophenyl)-4,5-dihydro-1H-5-pyrazolone (ALIPD28):

The product was recrystallized with absolute ethanol. Beige solid. Yield: 70 %. mp: 199°C. Rf (cyclohexane / ethyl acetate 8:2): 0,78. ¹H NMR (DMSO): 1,71-2,00 (m, 15H); 5,43 (s, 1H); 7,41 (s, 1H); 7,81 (s, 2H); 12,02 (s, 1H). LC-MS (ESI⁺): 363,2 (MH⁺); tᵣ= 4,23 min.

### 1-(2-pyridyl)-3-[3-(trifluoromethyl)phenyl]-4,5-dihydro-1H-5-pyrazolone (ALIPD58):

The precipitate obtained was filtered and washed with ethanol. Grey solid. Yield: 50 %. mp: 108 °C. Rf (cyclohexane / ethyl acetate 5:5): 0,37. ¹H NMR (CDCl₃): 5,90 (s, 1H); 7,13-7,23 (m, 2H); 7,51-7,62 (m, 1H); 8,00-8,02 (m, 1H); 8,06-8,09 (d, 1H, *J*=8,3 Hz); 8,16 (s, 1H); 8,30-8,32 (d, 1H, *J*=4,4 Hz); 12,79 (s, 1H). LC-MS (APCl⁺): 306 (MH⁺); tᵣ=3,49 min.

### 1-(2,4-dichlorophenyl)-3-(4-methoxyphenyl)-4,5-dihydro-1H-5-pyrazolone (ALIPX14):

The product was purified by chromatography on silica gel with the eluent: cyclohexane / ethyl acetate (8:2). Brown solid. Yield: 38 %. mp: 126-127 °C. Rf (cyclohexane / ethyl acetate 6:4): 0,73. ¹H NMR (CDCl₃): 3,87 (s, 3H); 5,94 (s, 1H); 6,79 (d, 1H, *J*=8,4 Hz); 6,97 (d, 2H, *J*=9 Hz); 7,22 (d, 1H, *J*=8,4 Hz); 7,61 (s, 1H); 7,85 (d, 2H, *J*=9 Hz). LC-MS (APCI⁺): 335 (MH⁺); tᵣ= 3,94 min.

### 1-(2,5-Dichlorophenyl)-3-(phenyl)-4,5-dihydro-1H-5-pyrazolone (ALIPD48):

The product was recrystallized with acetonitrile. White solid. Yield: 42 %. mp: 218 °C. Rf (cyclohexane / ethyl acetate 8:2): 0.34. 1H NMR (DMSO): 5,96 (s, 1H); 7,03-7,40 (m, 3H); 7,57 (m, 2H); 7,74-7,77 (m, 2H); 7,85 (m, 1H) 11,57 (s,1H). LC-MS (ESI+): 305,1 (MH+); tr= 2,93 min.

### 1-(3,5-Dichlorophenyl)-3-(4-methoxyphenyl)-4,5-dihydro-1H-5-pyrazolone (ALIPX02):

The product was purified by chromatography on silica gel with the eluent: cyclohexane / ethyl acetate (1:1). Orange solid. Yield: 46%. mp: 193-194°C. Rf (cyclohexane / ethyl acetate): 1,81. ¹H NMR (CDCI₃): 3,85 (s, 2H); 3,88 (s, 3H); 6,98 (d, 2H, *J*=8,7 Hz); 7,19 (s, 1H); 7,72 (d, 2H, *J*=9 Hz); 8,01 (s, 2H). LC-MS (APCl⁺): 334,9 (MH⁺); tᵣ= 4,62 min.

### 3-(4-chlorophenyl)-1-(2-pyridyl)-4,5-dihydro-1H-5-pyrazolone (ALIPD59):

The precipitate obtained was filtered, and washed with ethanol (Fraction 1: yield 69%). The filtrate was recovered and concentrated at reduced pressure. A solid was obtained which was recrystallized with ethanol (Fraction 2: yield 26%). Grey solid. Yield: 95 %. mp: 113 °C. Rf (cyclohexane / ethyl acetate 5:5): 0,62. 1H NMR (CDCl3): 5,94 (s, 1H); 7,19-7,23 (m, 1H); 7,39-7,43 (m, 2H); 7,80-7,84 (m, 2H); 7,90-7,96 (m, 1H); 8,04-8,07 (d, 1H, J=8,4 Hz); 8,30-8,32 (d, 1H, J=5 Hz); 12,82 (s, 1H).). LC-MS (ESI+): 272 (MH+); tr=3,45 min.

### 1-(2,4-Dichlorophenyl)-3-(4-methoxyphenyl)-4,5-dihydro-1H-5-pyrazolone (ALIPX05):

The product was purified by chromatography on silica gel with the eluent: cyclohexane / ethyl acetate (9:1). White solid. Yield: 31 %. mp: 186-187 °C. Rf (cyclohexane / ethyl acetate 6:4): 0,46. ¹H NMR (CDCl₃): 3,81 (s, 2H); 3,87 (s, 3H); 6,95 (d, 2H, *J*=9 Hz); 7,37 (d, 1H, *J*=2 Hz); 7,43 (s, 1H); 7,54 (d, 1H, *J*=2,3 Hz); 7,66 (d, 2H, *J*=8,7 Hz). LC-MS (APCl⁺): 335,1 (MH⁺); tᵣ= 3,91 min.

### 1-(2,4-Dichlorophenyl)-3-(4-nitrophenyl)-4,5-dihydro-1H-5-pyrazolone (ALIPD33):

The product was recrystallized with acetonitrile. Beige solid. Yield: 39 %. mp: 201°C. Rf (cyclohexane / ethyl acetate 8:2): 0,26. 1H NMR (DMSO): 6,17 (s, 1H); 7,60 (dd, 1H, J=8,5 Hz J=2,0 Hz); 7,61 (d, 1H, J=8,5 Hz); 7,88 (dd, 1H, J=1,9 Hz, J=0,6 Hz); 8,05 (d, 1H, J=2,3 Hz); 7,26 (d, 2H, J=8,7 Hz); 11,88 (s,1H). LC-MS (ESI-): 348,1 (MH-); tr= 3,02 min.

### 1-(2,5-Dichlorophenyl)-3-(cycloheptyl)-4,5-dihydro-1H-5-pyrazolone (ALIPD38):

The product was purified by chromatography on silica gel with the eluent: cyclohexane / ethyl acetate (8:2). The product was recrystallized with cyclohexane. Beige solid. Yield: 7 %. mp: 139°C. Rf (cyclohexane / ethyl acetate 8:2): 0,26. ¹H NMR: (CDCl₃): 1,55-1.99 (m, 12H); 2.63-2.69 (m, 1H); 3,40 (s, 2H); 7,33 (dd, 1H, *J*=8,5 Hz, *J*=2,1 Hz); 7,36 (d, 1H, *J*=8,5 Hz); 7,51 (d, 1H, *J*=2,1 Hz). LC-MS (ESI⁺): 325,2 (MH⁺); tᵣ= 3,22 min.

### Microscale thermophoresis (MST)

### Requirements

The quantity of cells for this protocol can vary from 10 to 40 million. The optimal fluorescence of lysate is situated between 200 and 1 600 units of fluorescence (Fl Units). Different types of additives such as Tween 20, various MgCl₂ concentrations, BSA have been tested to avoid sample aggregation and different types of capillaries have been investigated to reduce the adsorption within capillaries. Various buffers have also been tested and are listed in the related section. The fluorescence measurement is made using a small quantity of cell lysate within a capillary chosen among different available capillaries: standard, premium, hydrophilic or hydrophilic. The highest concentration of ligand must be at least 20 times higher than the expected dissociation constant of the interaction under study. However, the lowest concentration of ligand must be lower than the concentration of the fluorescent protein. An online tool created by NanoTemper® can be used and allows determining the range of ideal concentration. The concentration of the fluorescent protein can be determined using a GFP dosage kit (Abnova KA0911) or a calibration curve.

### Cell transfection

CHO-K1 cells were transfected with the Cell Line Nucleofector® Kit T, Program U-023 and the pcDNA 3.1 hygro plasmid expressing PD-1-eGFP. 2 µg of plasmid was transfected in one million CHO-K1 cells suspended in 100 µL Nucleofector solution. Cells were analyzed 48 hours post Nucleofection® by flow cytometry. Cells are put under the appropriate selective pressure which will only allow growth of cells containing the DNA insert. After three weeks of selective pressure, the multiparametric analysis of transfected CHO-K1 cells over-expressing mPD-1-eGFP allows defining sub-populations which can be separated from the global population. The population of cells strongly expressing eGFP is analyzed by flow cytometry and kept for MicroScale Thermophoresis analysis. The exact same protocol from transfection to cytometry analysis is lead on CHO-K1 cells expressing the hPD-L1-eGFP protein.

### PD-L1 binding assay

MST was used to establish the binding affinity between PD-L1-eGFP and Compounds. Cell lysate was diluted in PBS-T buffer (0.05% Tween 20) to a final concentration at which the fluorescent signals of the GFP proteins were similar and above the typical detection limit of the Monolith NT.115 instrument (NanoTemper Technologies). The final concentration of cell lysate was kept constant to 35 nM. For the binding between hPD-L1-eGFP with compounds, several dilutions were made between 1mM to 1 nM to cover a wide concentration range. Ten microliters of cell lysate are added to 10 µL of each ligand solution. Nivolumab was taken as reference.

### FRET assay

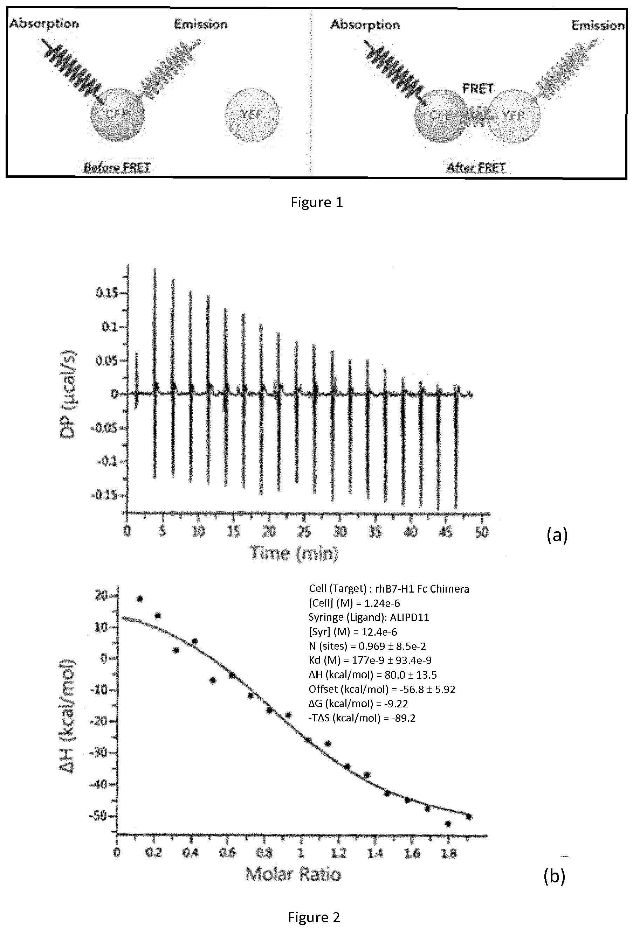

Energy transfer between fluorescent molecules (FRET) is a technique that allows visualizing interactions between 2 fluorescent molecules. A fluorescent molecule, called "donor", will transfer its energy, once excited, to another fluorescent molecule called "acceptor". The fluorescence of the donor decreases while the acceptor's one increases (see Figure 1).

A model was developed using CHO-K1 cells involving overexpression of proteins of interest. It involves the expression of a PD-1-YFP fluorescent protein and a second fluorescent protein SHP-2-CFP (phosphatase recruited during the interaction between PD-1 and PD-L1).

During excitation at a given length (λ_{CFP} 445-485 nm, λ_{YFP} 485-535 nm), an energy transfer takes place when the proximity is sufficient between the two proteins resulting in a FRET phenomenon. Using a blocking compound, the interaction is inhibited. Therefore, the recruitment of phosphatase no longer intervenes. The FRET phenomenon is therefore no longer observed.

Molecules have been evaluated to determine their abilities as drug candidates. Several controls were carried out.

### Protocol

A Spectramax i3 is configured according to the selected fluorochromes with their excitation and emission spectra. Endpoint reading is performed, at the center of the well; the reading time is 2 minutes for a 96-well plate.

For this test, a 96-well flat-bottomed white plate is required. All mixes introduced into the plate are made of triplicate. The negative control is provided by the CHO transfected by the void plasmid, the positive control is provided by CHO transfected with a plasmid containing the YFP-CFP fusion protein making it possible to mimic the FRET phenomenon. To check that the two fluorochromes do not emit in the FRET channel separately, the PD-1-YFP and SHP2-CFP constructs are checked separately. It is also necessary to remove the background noise, that is to say the FRET without activation with PD-L1. After the addition of PD-L1 (10 µM) to our co-transfected PD-1-SHP2 cells, dose-response curved are performed to determine the IC₅₀ of the molecule under study. The IC₅₀ values are obtained using Graphpad and the results are listed above. Nivolumab was taken as positive control of interaction's inhibitions.

### ITC

ITC was used to study the binding of the compounds of the invention. Interactions between biomolecules or macromolecules like proteins, nucleic acids, or lipids are essential for all cellular processes. The characterization of interactions between biomolecules is thus an important task in basic and applied life science, as well as in the pharmaceutical and biotech industry. Every interaction between two molecules generates or absorbs heat, depending on the thermodynamic nature of the interaction. This heat change is measured in Isothermal Titration Calorimetry (ITC) by titrating one interaction partner (usually present in a syringe) to the other interaction partner (usually present in a reaction cell). From the raw heat pulses, binding isotherms are derived that deliver comprehensive information of the affinity, stoichiometry, and thermodynamics of molecular interactions.

Principle: ITC directly measures the heat released or absorbed during molecular binding events and the concomitant formation of molecular complexes. The label-free, in-solution characteristic of ITC experiments allows for the direct and native-like determination of all important parameters that characterize the thermodynamics of a molecular interaction: the binding constant (K_{D}), the reaction stoichiometry (n), the observed binding enthalpy (ΔH_{obs}), the observed binding entropy (ΔS_{obs}), the observed heat capacity of binding (ΔC_{obs}) and finally the change in free enthalpy of binding (ΔG). Thus, ITC generates a complete thermodynamic profile of a molecular interaction and, for example, can help to differentiate between binding reactions that are driven by enthalpy (due to the formation of non-covalent interactions across the binding-interface) or by entropy (due to the release of water molecules from a binding pocket).

### Material and methods

**Table 2: Conditions of the ITC assays**

| Assay type ITC interaction analysis | |
|---|---|
| Target (in the ITC cell) | rhB7-H1 Fc Chimera |
| Target assay concentration | 1.24 µm |
| Target buffer | 1xPBS pH 7.4 0.5% DMSO |
| Ligands (in the ITC syringe) | ALIPD11 |
| Ligand assay concentration | 12.4 µm |
| Ligand buffer | 1xPBS pH 7.4 0.5% DMSO |
| Technical settings | Reference power 10 µcal/s, Feedback high, Stirring 750 rpm, Spacing 150s |
| Assay buffer | 1xPBS pH 7.4 0.5% DMSO |
| Temperature | 25 °C |
| Controls | For each ligand, a control titration of ligand into assay buffer was performed |

All samples were stored at -80°C prior to the experiment phase. The target samples were prepared as follows: the target stock solution was diluted to the final assay concentration and in a way to reach the target buffer condition. The final target assay solution was loaded into the reaction cell of the ITC device.

The ligand samples were prepared as follows: the ligand stock solution was diluted to the final assay concentration and in a way to reach the ligand buffer condition. The final assay solution was filled into the titration syringe of the ITC device.

The experiments were performed on a Malvem PEAQ-ITC device. In order to control the device integrity, a control experiment was performed in which water was titrated against water. All data were analyzed using the corresponding PEAQ -ITC.

### Results

### • rhB7-H1 Fc Chimera vs ALIPD11

The raw heat plot (see Figure 2a) shows titration peaks with an overall signal spread of 0.3 µcal/s. The enthalpy plot (see Figure 2b) indicates an interaction between ligand ALIPD11 and the target. The obtained data are fitted and the following main interaction parameters are obtained:

**Table 3: Results of the ITC assay**

| Parameters | Value |
|---|---|
| N (reaction stoichiometry) | 0.97±0.09 (indicating 1:1 interaction) |
| K_{D} (steady state affinity) | 177±93 nM |
| ΔG(free enthalpy of binding) | -0.922 kcal/mol (indicating exergonic reaction) |

### CFSE Cell Proliferation Kit, for flow cytometry

The CellTrace™ Cell Proliferation Kits provide versatile and well-retained cell tracing reagents in a convenient and easyto-use form. Each kit contains a CellTrace™ reagent in single-use vials to permit small scale experiments without preparing excess quantities of stock solution. The CellTrace™ reagents readily diffuse into cells and bind covalently to intracellular amines, resulting in stable, well-retained fluorescent staining that can be fixed with aldehyde fixatives. Excess unconjugated reagent passively diffuses to the extracellular medium, where it can be quenched with complete media and washed away. The kit is supplied by ThermoFisher Scientific.

### Protocol

1. Number of days on which the manipulation is done: 3 (from D0 to D3: 4 passages in cytometry)
2. Volume of cell suspension needed: 2.5ml (0.5ml / day)
3. Number of cells needed: 2.5mlx0.5M / ml = 1.25M per cell line.
4. Number the cells and check their viability.
5. Prepare the desired number of cells.
6. Centrifuge, resuspend at a 1M/mL concentration in PBS (the probe binds to the proteins of the medium)
7. Add 0.5µM of CellTrace (dilute 1/10 of the stock solution, add 1 / 1000th in the PBS-cell mix: take 1µl of probe at the main concentration, add 9µL of PBS)
8. Incubate for 20 minutes at 37 ° C in the dark.
9. Add 5 volumes of medium to capture the excess of probe (1.2x5 = 6ml)
10. Incubate for 5 minutes at 37 ° C.
11. Centrifuge, resuspend the cells at the desired concentration of 0.5M / ml in preheated medium. Plate 24 wells.
12. Establish the J0 at least 5h after marking.
13. Flow cytometry is then performed to evaluate cell division and to determine an IC50 of proliferation using Graphpad.

### Conclusion

In summary, the molecules of the present invention, and in particular AliPD11, AliPD19, AliPD51, AliPD55 and AliPD60, have a real therapeutic interest in the treatment of cancers. They have an affinity (Kd) of the order of nM, anaffinity which is comparable to that of Nivolumab, an antibody used in clinical use. Indeed, molecules AliPD11, AliPD19, AliPD51, AliPD55 and AliPD60 have a respective Kd of 19nM, 83nM, 45nM, 7nM and 23nM compared to Nivolumab which has a Kd of 20 nM.

This affinity is correlated with in vivo activity for the molecules AliPD11, AliPD19, AliPD51, AliPD55 and AliPD60 resulting in a better IC50 (9nM, 17nM, 44nM, 3nM and 7nM) than that observed with Nivolumab(14nM) in the FRET test.

The molecules of the present invention, an in particular AliPD11, AliPD19, AliPD51, AliPD51, AliPD55 and AliPD60 therefore have the ability due to their affinity for PD-L1 and their ability to block the PD1/PD-L1 interaction to restore the anti-tumor immune response.

For this reason, the molecules of the present invention represent a good therapeutic for the treatment of cancers.

## Claims

1. Compound of the following formula (I): 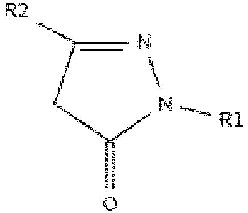 or a pharmaceutically acceptable salt and/or solvate,
wherein
R¹ is selected in the group consisting of: 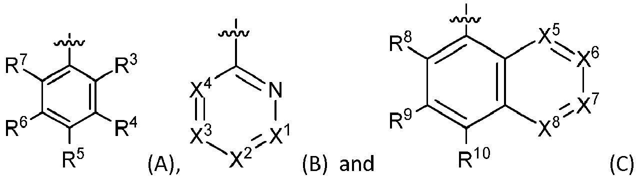
X¹ is N or CR¹¹, X² is N or CR¹², X³ is N or CR¹³, X⁴ is N or CR¹⁴, X⁵ is N or CR¹⁵, X⁶ is N or CR¹⁶, X⁷ is N or CR¹⁷ and X⁸ is N or CR¹⁸, provided that at least two of X¹ to X⁴ are not N and that at least two of X⁵ to X⁸ are not N,
R³ to R¹⁸ are each independently selected in the group consisting of H, halogen, such as Cl or F, CN, COR¹⁹ and OR²⁰, R¹⁹ and R²⁰ being each independently H or C₁-C₆ alkyl,
R² is selected in the group consisting of carbocyclyl, heterocyclyl, aryl and heteroaryl, R² being optionally substituted by one or more groups selected in the group consisting of halogen, such as Cl or F, NO₂, C₁-C₆ haloalkyl, such as CF₃, oxo, CN, SO₂ and OR²¹, R²¹ being H or C₁-C₆ alkyl.

2. The compound according to claim 1, wherein R³ to R¹⁸ are each independently selected in the group consisting of H, halogen, and OR²⁰, R²⁰ being preferably C₁-C₆ alkyl such as a methyl.

3. The compound according to claim 1 or 2, wherein R¹ is of formula (A) and R3 to R7 are preferably selected in the group consisting of H, Cl and OCH₃.

4. The compound according to claim 1 or 2, wherein R¹ is of formula (B).

5. The compound according to claim 4, wherein X¹ is CR¹¹, X² is CR¹², X³ is CR¹³ and X⁴ is CR¹⁴, R¹¹ to R¹⁴ being preferably H.

6. The compound according to claim 1 or 2, wherein R¹ is of formula (C),

7. The compound according to claim 6, wherein one or two, preferably one, of X⁵ to X⁸ are N.

8. The compound according to any one of claims 1 to 7, wherein R² is selected in the group consisting of carbocyclyl and aryl, such as phenyl, R² being optionally substituted by one or more groups, preferably one or two, selected in the group consisting of halogen, such as Cl or F, NO₂, C₁-C₆ haloalkyl, such as CF₃, oxo and OR²¹, R²¹ being preferably C₁-C₆ alkyl, such as methyl.

9. The compound according to any of claims 1 to 8, wherein R² corresponds to a carbocyclyl, preferably of the following formula:

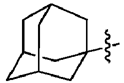

10. The compound according to any one of claims 1 to 9 being selected from the group consisting of: 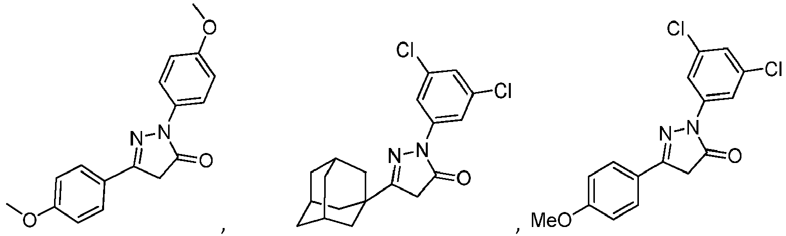 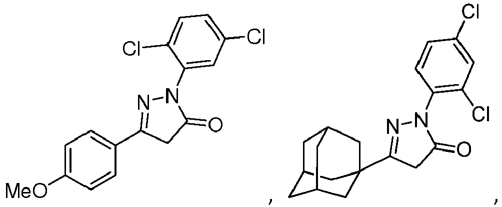 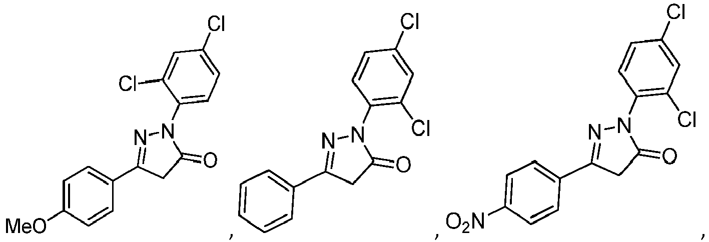 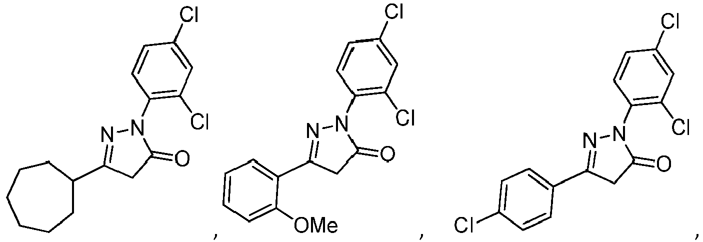 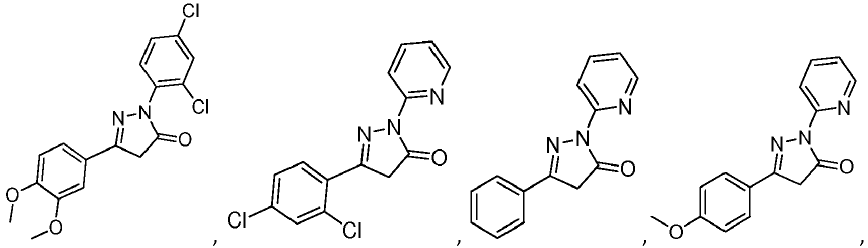 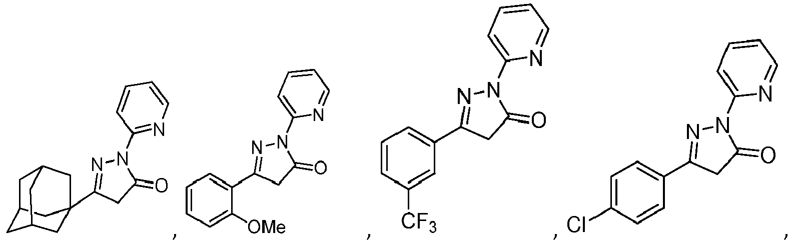 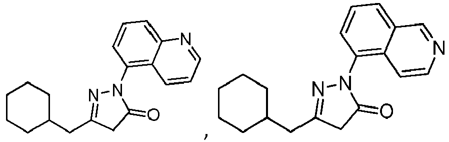 preferably 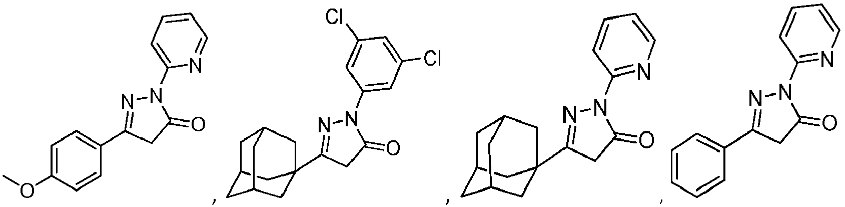 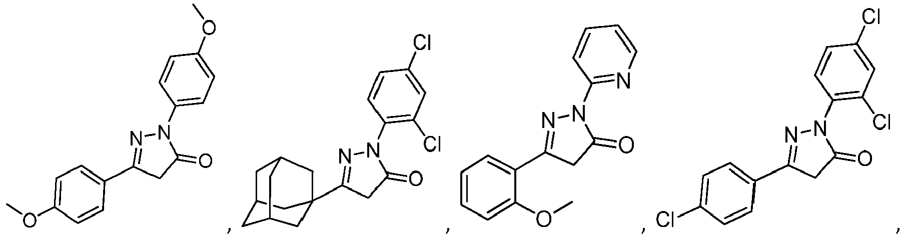 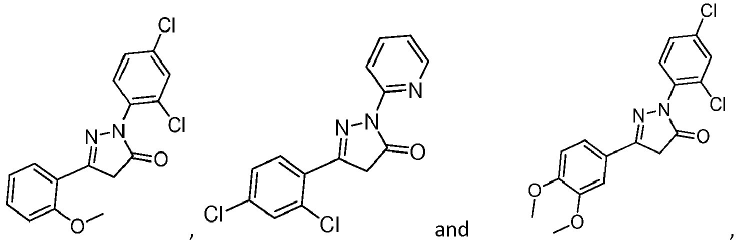 more preferably 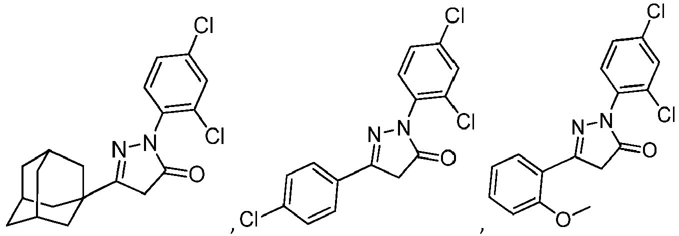 and

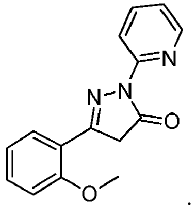

11. A pharmaceutical composition comprising at least one compound according to any one of claims 1 to 10 and at least one pharmaceutically acceptable excipient.

12. The compound according to any one of claims 1 to 10 or the composition according to claim 11 for use as a drug.

13. The compound or the composition for use according to according to claim 12 for use in the prevention and/or treatment of PD-L1-PD-1 interactions-related diseases, such as cancer, neurological diseases and chronic infections.

14. The compound or the composition for use according to claim 13, wherein cancer is selected from lung carcinoma (Non small cell and Small cell), head and neck carcinoma, bladder carcinoma, kidney carcinoma, triple negative breast cancer, melanoma, gastric carcinoma, oesophagus carcinoma, Hodgkin's lymphoma, non Hodgkin lymphoma, glioblastoma, multiple myeloma, acute myeloide leukemia, Cholangiocarcinoma (gallbladder carcinoma), Merkel carcinoma, squamous cell carcinoma and endometrial carcinoma, neurological diseases is Alzheimer disease and chronic infections is selected from HIV, malaria, tuberculosis and B hepatitis.

15. A method for the preparation of a compound according to any one of claims 1 to 10 comprising reacting a β-cetoester derivative of the following formula (II): 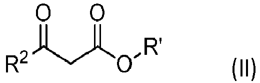 wherein R² is as defined in claim 1 and R' is a C₁-C₆ alkyl, preferably an ethyl,
with a hydrazine hydochloride derivative of the following formula (III): 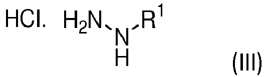 in presence of a base, in particular sodium acetate, advantageously in a polar solvent.
